# EUROPÄISCHE PATENTSCHRIFT

(11) **EP 0 607 804 B1**
(45) Veröffentlichungstag und Bekanntmachung des Hinweises auf die Patenterteilung: **10.07.1996**
(21) Anmeldenummer: 94100107.5
(22) Anmeldetag: 05.01.1994
(51) Int. Cl.: C07D 213/73

(54) **Verfahren zur Herstellung von 2-Amino-5-aminomethyl-pyridin**
Process for the preparation of 2-amino-5-aminomethylpyridine
Procédé pour la préparation de 2-amino-5-aminométhylpyridine

(30) Priorität: 18.01.1993 DE 4301110
(43) Veröffentlichungstag der Anmeldung: 27.07.1994
(73) Patentinhaber: BAYER AG, 51368 Leverkusen (DE)
(72) Erfinder: Kraus, Helmut, Dr., D-51519 Odenthal (DE)

(56) Entgegenhaltungen:
- EP-A- 0 060 071
- SCIENCE Bd. 166, Nr. 3901 , 3. Oktober 1969 , LANCASTER, PA US Seiten 765 - 766 J.P. FERRIS ET AL. 'Photochemical reactions and the chemical evolution of purines and nicotinamide derivatives'
- ERWIN KLINGSBERG (EDITOR) 'Pyridine and its derivatives Part three' 1962 , INTERSCIENCE PUBLISHERS , NEW YORK

## Beschreibung

Die Erfindung betrifft ein Verfahren zur Herstellung von 2-Amino-5-aminomethyl-pyridin durch Umsetzung von Methylen-glutaconsäuredinitrilen mit Ammoniak und Wasserstoff in Gegenwart eines Hydrierkatalysators in einer "Eintopf"-Reaktion, die gegebenenfalls in einem Lösungsmittel als Reaktionsmedium durchgeführt werden kann, 2-Amino-5-aminomethyl-pyridin (AAMP) ist ein wichtiges Zwischenprodukt zur Synthese von Insektiziden der Nitromethylenklasse (EP 163 855, EP 376 279).

Die Herstellung geeigneter 2,5-disubstituierter Pyridine, aus denen die obigen Schlüsselverbindungen zugänglich sind, ist technisch schwierig. So ist 6-Chlornicotinsäure, deren Seitenkette weiter funktionalisiert werden müßte, nur durch bakterielle Hydroxylierung und anschließende Chlorierung zugänglich (EP 152 949, EP 72 777), 2-Chlor-5-methylpyridin, dessen Methylgruppe schwierig zu chlorieren ist, kann durch Halogenierung des Picolin-N-oxids (DE-OS 38 39 332) oder aus 2-Amino-5-methylpyridin (DE-AS 16 95 659) herge-stellt werden. Die Halogenierung des N-Oxids und die Aminierung von β-Picolin sind jedoch jeweils mit dem Problem der Bildung unerwünschter Isomerer behaftet, was zu technisch aufwendigen Verfahren und lästigen Trenn-operationen führt. Weiterhin kann 2-Amino-5-phenyl-pyri-din, ausgehend von 4-Dimethylamino-3-phenyl-pentadien-(1,3)-nitril mit Ammoniak in Dimethylsulfoxid hergestellt werden (EP-A-60 071); jedoch erhält man hierbei aus 3 g Ausgangsmaterial nur 300 mg des gewünschten Produktes. 6-Amino-nicotinsäure-nitril ist durch Belichten von 2-Formyl-glutaconsäuredinitril in 1-molarer ammoniakalischer Lösung in 10%iger Ausbeute zugänglich (Science 166 (1969), 765).

Bei Betrachtung dieses Standes der Technik war es überraschend, daß AAPM, ausgehend von einem leicht zugänglichen aliphatischen C₆-Baustein isomerenfrei und technisch einfach in hoher Ausbeute hergestellt werden kann. Das bei der Cyclisierung entstehende Spaltmolekül, beispielsweise ein sekundäres Amin, führt überraschenderweise zu keiner Beeinträchtigung der Reaktionsführung. Trotz hoher Nucleophilie eines solchen sekundären Amins gegenüber Ammoniak liegt der Anteil an tertiärem Amin deutlich unterhalb der statistisch zu erwartenden Mengen.

Die Erfindung betrifft demnach ein Verfahren zur Herstellung von 2-Amino-5-aminomethyl-pyridin der Formel 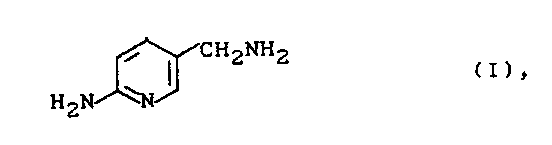 das dadurch gekennzeichnet ist, daß man ein substituiertes Methylen-glutaconsäuredinitril der Formel 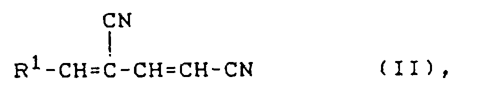 in der
- R¹: für -OR² oder -N(R²,R³) steht,
worin R² und R³ unabhängig voneinander geradkettiges oder verzweigtes C₁-C₈-Alkyl, C₃-C₈-Alkenyl, C₂-C₈-Alkoxyalkyl, C₄-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R² und R³ mit dem N-Atom, das sie substituieren, einen 5- bis 8-gliedrigen Ring bilden können, der ein weiteres Heteroatom aus der Gruppe von N, O und S enthalten kann,
bei 50 bis 200°C, bevorzugt 60 bis 150°C zunächst mit 3 bis 200 Mol, bevorzugt 5 bis 100 Mol Ammoniak und dann in Gegenwart eines Hydrierkatalysators mit Wasserstoff, der mit einem H₂-Partialdruck von 10 bis 250 bar, bevorzugt 20 bis 200 bar vorliegt, umsetzt, wobei mit oder ohne Lösungsmittel gearbeitet werden kann.

Geradkettiges oder verzweigtes C₁-C₈-Alkyl ist beispielsweise Methyl, Ethyl, Propyl, Isopropyl, Butyl, Isobutyl, tert.-Butyl, die isomeren Pentyle, Hexyle, Octyle, bevorzugt die genannten C₁-C₄-Alkylreste.

Geradkettiges oder verzweigtes C₃-C₈-Alkenyl ist beispielsweise Allyl, die isomeren Butenyle, Pentenyle, Hexenyle oder Octenyle, bevorzugt die genannten C₃-C₄-Alkenylreste.

Geradkettiges oder verzweigtes C₂-C₈-Alkoxyalkyl ist beispielsweise Methoxymethyl, Ethoxymethyl sowie weitere Reste aus der Gruppe C₃-C₉-Alkyl, in welchem eine CH₂-Gruppe durch ein O-Atom ersetzt ist.

Geradkettiges oder verzweigtes C₄-C₈-Alkoxyalkenyl ist beispielsweise Methoxyallyl, 2-Methoxy-propenyl und andere aus der Gruppe von C₄-C₉-Alkenyl, worin eine CH₂-Gruppe durch ein O-Atom ersetzt ist.

C₃-C₈-Cycloalkyl ist beispielsweise Cyclopropyl, Methyl-cyclopropyl, Dimethyl-cyclopropyl, Cyclobutyl, Methyl-cyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl, sowie deren Methyl- oder Dimethyl-Derivate.

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenylethyl, 2-Phenylethyl und weitere dem Fachmann bekannte Reste dieser Art, bevorzugt Benzyl.

Als 5- bis 8-gliedriger gesättigter oder ungesättigter heterocyclischer Ring, dessen Heteroatome 1 oder 2 aus der Gruppe von N, O und S sind, seien genannt: Pyrrol, Furan, Thiophen, Pyrrolidin, Pyrazol, Imidazol, Thiazol, Oxazol, Pyridin, Pyrimidin, Piperazin, das am N-Atom durch C₁-C₄-Alkyl oder durch Hydroxy-C₁-C₄-alkyl substituiert sein kann, Morpholin, Pyran, Azepin, Azocin, Isoxazol, Isothiazol, Pyridazin und Pyrazin. Es ist dem Fachmann bekannt, daß ungesättigte heterocyclische Ringe einen mehr oder weniger stark ausgeprägten aromatischen Charakter haben können, In bevorzugter Weise seien als solche gesättigte oder ungesättigte heterocyclische Ringe Morpholin, Pyrrolidin und Piperidin, das durch C₁-C₄-Alkyl oder durch Hydroxy-C₁-C₄-alkyl substituiert sein kann, genannt.

Weiterhin können R² und R³ gemeinsam mit dem N-Atom, das sie substituieren, einen 5- bis 8-gliedrigen gesättigten oder ungesättigten Ring bilden, der ein weiteres Heteroatom aus der Gruppe N, O und S enthalten kann. Solche Ringe sind beispielsweise die oben genannten Heterocyclen.

In bevorzugter Weise werden im erfindungsgemäßen Verfahren Enamine eingesetzt, in denen an die Stelle von R², R³ die Substituenten R¹², R¹³ treten, die unabhängig voneinander geradkettiges oder verzweigtes C₁-C₈-Alkyl, Cyclopropyl, Cyclopentyl, Cyclohexyl, Phenyl oder Benzyl bedeuten, wobei weiterhin R¹² und R¹³ gemeinsam mit dem N-Atom, das sie substituieren, einen 5- bis 8-gliedrigen Ring bilden können, der ein weiteres Heteroatom aus der Gruppe von N, O und S enthalten kann.

In besonders bevorzugter Weise werden im erfindungsgemäßen Verfahren Enamine eingesetzt, in denen an die Stelle von R¹², R¹³ die Substituenten R²², R²³ treten, 0 die unabhängig voneinander C₁-C₄-Alkyl bedeuten, wobei weiterhin R²² und R²³ gemeinsam mit dem N-Atom, das sie substituieren, Morpholin, Pyrrolidin oder Piperidin, das durch C₁-C₄-Alkyl oder durch Hydroxy-C₁-C₄-alkyl substituiert sein kann, bedeuten.

In bevorzugter Weise tritt an die Stelle von R¹ der Substituent R¹¹ mit der Bedeutung -N(R²,R³), wobei R² und R³ unabhängig voneinander die weiter oben gegebene Bedeutung haben.

Die Umsetzung des erfindungsgemäßen Verfahrens kann in überschüssigem Ammoniak durchgeführt werden. Zusätzlich kann ein Lösungsmittel als Reaktionsmedium eingesetzt werden. Als Lösungsmittel kommen einzelne oder ein 5 Gemisch mehrerer aus den Gruppen Kohlenwasserstoffe, halogenierte Kohlenwasserstoffe, tertiäre Amine, Ketone, Nitrile, Dialkylcarboxamide, Ether, Phosphorsäureperalkylamide, Sulfonsäuredialkylamide, N-Alkyl-lactame, Peralkyl-harnstoffe, Dialkyl-sulfoxide, Dialkylsulfone, 0 Alkohole in Frage. Einzelbeispiele solcher Lösungsmittel, deren Aufzählung keineswegs erschöpfend ist, sind: Petrolether, Toluol, Xylol, Cyclohexan, Chlorbenzol, Ligroin, Triethylamin, Anisol, Methyl-tert.-Dimethylformamid, Acetamid, N-Methyl-pyrrolidon, N-Methyl-caprolactam und Tetramethylharnstoff. Ein geringer Anteil Wasser im Reaktionsmedium stört nicht.

Die Reaktionstemperatur liegt im Bereich von 50 bis 200°C, bevorzugt bei 60 bis 150°C. Die Menge an Ammoniak beträgt 3 bis 200 Mol, bevorzugt 5 bis 100 Mol, bezogen auf das Methylen-glutaconsäuredinitril. Im oberen Teil dieses Bereiches wird man bevorzugt arbeiten, wenn ohne zusätzliches Lösungsmittel gearbeitet werden soll, und umgekehrt.

Wasserstoff liegt im Reaktionsgemisch überschüssig vor und zwar mit einem H₂-Partialdruck von 10 bis 250 bar, bevorzugt 20 bis 200 bar.

Die Reaktionszeit ist abhängig von verschiedenen Parametern, wie der Ansatzgröße, der Temperatur, dem Molverhältnis, dem Wasserstoffdruck und dem Hydrierkatalysator und liegt etwa bei 0,5 bis 10 Stunden, bevorzugt bei 0,5 bis 6 Stunden. Als Hydrierkatalysatoren eignen sich solche auf der Basis von Nickel und Kobalt sowie der Edelmetalle Pd, Pt und Rh, bevorzugt Ni und Co, die in einer dem Fachmann üblichen Weise mit Dotierungsstoffen, wie MgO, B₂O₃, TiO₂, V₂O₅, Cr₂O₃ und anderen versehen sein können. Solche Katalysatoren können auf Trägern angeordnet sein, wie SiO₂, Al₂O₃, Kohle, Zeolithe und weiteren dem Fachmann bekannten Trägern, Nickel- und Kobalt-Katalysatoren können jedoch auch ohne Träger, beispielsweise in Form der Skelett-Katalysatoren vom Raney-Typ, zur Anwendung kommen.

Das erfindungsgemäße Verfahren kann ohne Zwischenisolierung in einem Reaktor durchgeführt werden. Hierbei wird das Methylen-glutaconsäuredinitril zunächst mit Ammoniak, gegebenenfalls in Gegenwart eines der genannten Lösungsmittel, umgesetzt und danach mit Wasserstoff weiter behandelt. Der Hydrierkatalysator kann zwischen der Umsetzung mit Ammoniak und der Weiterbehandlung mit Wasserstoff zugesetzt werden; er kann jedoch in handhabungstechnisch bevorzugter Weise bereits vor Beginn der Umsetzung mit Ammoniak zugesetzt werden.

Das erfindungsgemäße Verfahren kann beispielsweise wie folgt dargestellt werden: 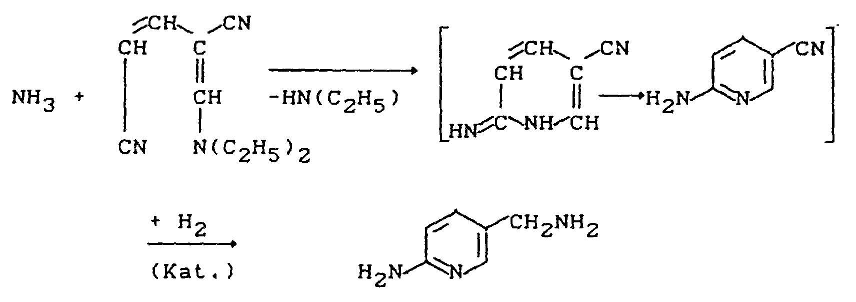 Diethylaminmethylenglutaconsäuredinitril reagiert mit Ammoniak unter Ringschluß zum Nicotinsäurenitril, welches anschließend zum 2-Amino-5-aminomethylpyridin hydriert wird.

Das eingesetzte Methylen-glutaconsäuredinitril ist beispielsweise durch Umsetzung von Glutaconsäuredinitril mit o-Estern bzw. o-Amiden erhältlich. Weiterhin können Aminomethylen-glutaconsäuredinitrile in technisch einfacher Weise durch Dimerisierung der zugrundeliegenden β-Amino-acrylnitrile erhalten werden.

### Beispiele

### Beispiel 1

15 g Dimethylaminomethylen-glutaconsäuredinitril, 75 ml Toluol und 3 g RaNi wurden in einem 0,3 l V4A-Autoklaven vorgelegt und 25 g Ammoniakgas aufgedrückt. Man heizte auf 100°C und nach 2 h wurden zusätzlich 100 bar Wasserstoff aufgedrückt (117 bar Gesamtdruck). Nach weiteren 5 h ließ man abkühlen und entspannte. Nach Zusatz von Methanol wurde das Reaktionsgemisch aus dem Autoklaven genommen, vom Katalysator abfiltriert und eingeengt.

Man erhielt 12,7 g 94,2 %iges Produkt, entsprechend 95,3 % der theoretischen Ausbeute.

Der Gehalt an 2-Amino-5-dimethylaminomethyl-pyridin betrug 1,3 %.
¹H-NMR (d-DMSO) 1,4-2,0 ppm (bs, 2H, NH₂), 3,2-3,6 ppm (s, 2H, NH₂), 5,68 ppm (s, 2H, CH₂), 6,39 ppm (d, 1H, H³), 7,38 ppm (dd, 1H, H⁴), 7,82 ppm (d, 1H, H⁶).

### Beispiel 2

15 g Glutaconsäuredinitril und 3 g RaNi wurden analog Beispiel 1 vorgelegt und 70 g Ammoniakgas aufgedrückt. Nach 2 h bei 100°C drückte man zusätzlich 100 bar H₂-Druck auf (Gesamtdruck: 112,8 bar) und hydrierte 4 1/2 h. Nach Abkühlen und Entspannen wurde mit Methanol aufgenommen, vom Katalysator abfiltriert und eingeengt. Man erhielt 12,5 g 95,9 %iges Produkt, entsprechend 95,6 % der theoretischen Ausbeute. Der Anteil an Dimethylaminoprodukt lag bei 0,6 %.

### Beispiel 3

Analog Bespiel 1 wurden 15 g Dinitril mit 30 ml Toluol und 10 g Ammoniak cyclisiert und hydriert. Die Ausbeute betrug 91,3 % der Theorie.

### Beispiel 4

15 g Diethylaminomethylen-glutaconsäuredinitril wurden mit 3 g RaNi und 30 g Ammoniak versetzt und bis zur Druckkonstanz bei 100°C hydriert. 2-Amino-5-amino-methylpyridin wurde in 93,7 % der Theorie erhalten.

### Beispiel 5

15 g Ethoxymethylen-glutaconsäuredinitril, 100ml Toluol und 3 g RaNi wurden vorgelegt und 30 g Ammoniakgas aufgedrückt. Nach weiterer Reaktionsführung analog Beispiel 1 wurde 2-Amino-5-aminomethylpyridin in 86,3 % der Theorie erhalten.

## Patentansprüche

1. Verfahren zur Herstellung von 2-Amino-5-amino-methyl-pyridin der Formel 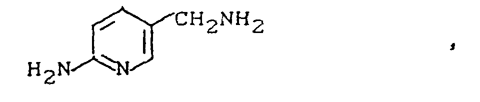 dadurch gekennzeichnet, daß man ein substituiertes Methylen-glutaconsäuredinitril der Formel 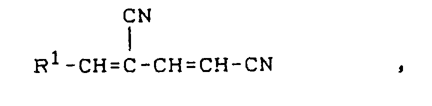 in der
R¹ für -OR² oder -N(R²,R³) steht,
worin R² und R³ unabhängig voneinander geradkettiges oder verzweigtes C₁-C₈-Alkyl, C₃-C₈-Alkenyl, C₂-C₈-Alkoxyalkyl, C₄-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R² und R³ mit dem N-Atom, das sie substituieren, einen 5- bis 8-gliedrigen Ring bilden können, der ein weiteres Heteroatom aus der Gruppe von N, O und S enthalten kann,
bei 50 bis 200°C, bevorzugt 60 bis 150°C zunächst mit 3 bis 200 Mol, bevorzugt 5 bis 100 Mol Ammoniak und dann in Gegenwart eines Hydrierkatalysators mit Wasserstoff, der mit einem H₂-Partialdruck von 10 bis 250 bar, bevorzugt 20 bis 200 bar vorliegt, umsetzt, wobei mit oder ohne Lösungsmittel gearbeitet werden kann.

2. Verfahren nach Anspruch 1, dadurch gekennzeichnt, daß an die Stelle von R¹ der Substituent R¹¹ mit der Bedeutung -N(R²,R³) tritt, wobei R² und R³ unabhängig voneinander die in Anspruch 1 gegebene Bedeutung haben.

3. Verfahren nach Anspruch 1, dadurch gekennzeichnet, daß an die Stelle von R² und R³ die Substituenten R¹² und R¹³ treten, die unabhängig voneinander geradkettiges oder verzweigtes C₁-C₈-Alkyl, Cyclopropyl, Cyclopentyl, Cyclohexyl, Phenyl oder Benzyl bedeuten, wobei weiterhin R¹² und R¹³ gemeinsam mit dem N-Atom, das sie substituieren, einen 5- bis 8-gliedrigen Ring bilden können, der ein weiteres Heteroatom aus der Gruppe von N, O und S enthalten kann.

4. Verfahren nach Anspruch 3, dadurch gekennzeichnet, daß an die Stelle von R¹² und R¹³ die Substituenten R²² und R²³ treten, die unabhängig voneinander C₁-C₄-Alkyl bedeuten und weiterhin gemeinsam mit dem N-Atom, das sie substituieren, Morpholin, Pyrrolidin oder Piperidin, das durch C₁-C₄-Alkyl oder durch Hydroxy-C₁-C₄-alkyl substituiert sein kann, bedeuten.

5. Verfahren nach Anspruch 1, dadurch gekennzeichnet, daß Lösungsmittel aus der Gruppe der Kohlenwasserstoffe, halogenierten Kohlenwasserstoffen, tert.-Aminen, Ketone, Nitrile, Dialkylcarboxamide, N-Alkyl-lactame, Peralkyl-harnstoffe, Dialkylsulfoxide, Dialkylsulfone, Ether, Alkohole, Phosphorsäureperalkylamide, Sulfonsäuredialkylamide stammen, und bevorzugt eines oder mehrere aus der Gruppe Petrolether, Toluol, Xylol, Cyclohexan, Chlorbenzol, Ligroin, Triethylamin, Anisol, Methyl-tert.-butylether, Dimethylformamid, Acetamid, N-Methyl-caprolactam, N-Methyl-pyrrolidon und Tetramethylharnstoff benutzt werden.

6. Verfahren nach Anspruch 1, dadurch gekennzeichnet, daß als Hydrierkatalysatoren Nickel- und/oder Kobalt-Katalysatoren bevorzugt als Raney-Ni bzw. Raney-Co, eingesetzt werden.

## Claims

1. Process for the preparation of 2-amino-5-aminomethyl-pyridine, of the formula 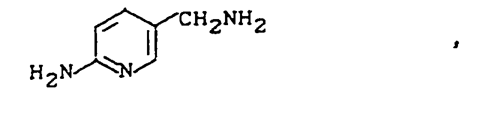 characterized in that a substituted methyleneglutaconic acid dinitrile of the formula 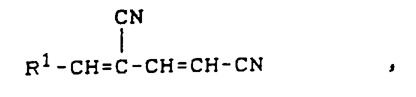 in which
R¹ represents -OR² or -N(R²,R³),
in which R² and R³, independently of one another, present straight-chain or branched C₁-C₈-alkyl, C₃-C₈-alkenyl, C₂-C₈-alkoxyalkyl, C₄-C₈-alkoxyalkenyl, C₃-C₈-cycloalkyl, C₆-C₁₂-aryl, C₇-C₁₀-aralkyl or a 5- to 8-membered saturated or unsaturated heterocyclic ring containing 1 or 2 hetero atoms from the group consisting of N, O and S, in which case R² and R³ may in addition, together with the N atom on which they are substituents, form a 5- to 8-membered ring which may contain a further hetero atom from the group consisting of N, O and S,
is initially reacted at from 50 to 200°C, preferably from 60 to 150°C, with from 3 to 200 mol, preferably from 5 to 100 mol, of ammonia and then reacted in the presence of a hydrogenation catalyst with hydrogen which is present at an H₂ partial pressure of from 10 to 250 bar, preferably from 20 to 200 bar, the reaction being carried out with or without solvent.

2. Process according to Claim 1, characterized in that the place of R¹ is taken by the substituent R¹¹ having the meaning -N(R²,R³), in which R² and R³, independently of one another, have the meaning given in Claim 1.

3. Process according to Claim 1, characterized in that the place of R² and R³ is taken by the substituents R¹² and R¹³ which, independently of one another, denote straight-chain or branched C₁-C₈-alkyl, cyclopropyl, cyclopentyl, cyclohexyl, phenyl or benzyl, in which case R¹² and R¹³, together with the N atom on which they are substituents, may additionally form a 5- to 8-membered ring which may contain a further hetero atom from the group consisting of N, O and S.

4. Process according to Claim 3, characterized in that the place of R¹² and R¹³ is taken by the substituents R²² and R²³ which, independently of one another, denote C₁-C₄-alkyl and together with the N atom on which they are substituents, additionally denote morpholine, pyrrolidine or piperidine which may be substituted by C₁-C₄-alkyl or by hydroxy-C₁-C₄-alkyl.

5. Process according to Claim 1, characterized in that the solvents come from the group consisting of hydrocarbons, halogenated hydrocarbons, tertiary amines, ketones, nitriles, dialkylcarboxamides, N-alkyl-lactams, peralkyl-ureas, dialkyl sulphoxides, dialkyl sulphones, ethers, alcohols, phosphoric acid peralkylamides and sulphonic acid dialkylamides, and those preferably used are one or more from the group consisting of petroleum ether, toluene, xylene, cyclohexane, chlorobenzene, ligroin, triethylamine, anisole, methyl tert-butyl ether, dimethylformamide, acetamide, N-methylcaprolactam, N-methyl-pyrrolidone and tetramethylurea.

6. Process according to Claim 1, characterized in that the hydrogenation catalysts employed are nickel and/or cobalt catalysts, preferably as Raney Ni or Raney Co, respectively.

## Revendications

1. Procédé pour la préparation de la 2-amino-5-aminométhylpyridine de formule : 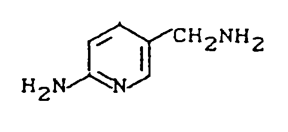 caractérisé en ce que l'on fait réagir un dinitrile méthylèneglutaconique substitué de formule: 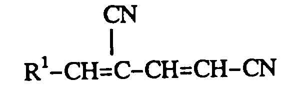 dans laquelle :
R¹ représente -OR² ou -N(R²,R³),
R² et R³ représentent chacun, indépendamment l'un de l'autre, un groupe alkyle en C₁-C₈ à chaîne droite ou ramifiée, alcényle en C₃-C₈, alcoxyalkyle en C₂-C₈, alcoxyalcényle en C₄-C₈, cydoalkyle en C₃-C₈, aryle en C₆-C₁₂, aralkyle en C₇-C₁₀ ou un hétérocycle saturé ou insaturé de 5 à 8 chaînons contenant un ou deux hétéroatomes choisis parmi N, O et S, R² et R³ pouvant également former avec l'atome d'azote dont ils sont substituants un cycle de 5 à 8 chaînons qui peut contenir un autre hétéroatome choisi parmi N, O et S,
à des températures de 50 à 200°C, de préférence de 60 à 150°C, d'abord avec 3 à 200 mol, de préférence 5 à 100 mol d'ammoniac puis, en présence d'un catalyseur d 'hydrogénation, avec l'hydrogène sous une pression partielle de 10 à 250 bar, de préférence de 20 à 200 bar, avec ou sans solvant.

2. Procédé selon la revendication 1 caractérisé en ce que, à la place de R¹, il y a un substituant R¹¹ consistant en un groupe -N(R²,R³) dans lequel R² et R³ ont chacun, indépendamment l'un de l'autre, l'une des significations indiquées dans la revendication 1.

3. Procédé selon la revendication 1 caractérisé en ce que, à la place de R² et R³, il y a des substituants R¹² et R¹³ qui consistent chacun, indépendamment l'un de l'autre, en un groupe alkyle à chaîne droite ou ramifiée en C₁-C₈, cyclopropyle, cyclopentyle, cyclohexyle, phényle ou benzyle, R¹² et R¹³ pouvant également former ensemble et avec l'atome d'azote dont ils sont substituants un cycle de 5 à 8 chaînons qui peut contenir un autre hétéroatome choisi parmi N, O et S.

4. Procédé selon la revendication 3 caractérisé en ce que, à la place de R¹² et R¹³, il y a des substituants R²² et R²³ qui consistent chacun, indépendamment l'un de l'autre, en un groupe alkyle en C₁-C₄ et peuvent en outre former ensemble et avec l'atome d'azote dont ils sont substituants un cycle morpholine, pyrrolidine ou pipéridine, lequel peut être substitué par un groupe alkyle en C₁-C₄ ou hydroxy-alkyle en C₁-C₄.

5. Procédé selon la revendication 1 caractérisé en ce que le solvant est choisi dans le groupe des hydrocarbures, des hydrocarbures halogénés, des amines tertiaires, des cétones, des nitriles, des dialkylcarboxamides, des N-alkyllactames, des peralkylurées, des dialkylsulfoxydes, des dialkylsulfones, des éthers, des alcools, des peralkylamides phosphoriques, des sulfodialkylamides et consiste de pré-férence en un ou plusieurs solvants du groupe de l'éther de pétrole du toluène, du xylène, du cyclohexane, du chlorobenzène, de l'essence minérale légère, de la tri-éthylamine, de l'anisole, de l'oxyde de méthyle et de tert-butyle, du diméthylformamide, de l'acétamide, du N-méthylcaprolactame, de la N-méthylpyrrolidone et de la tétraméthylurée.

6. Procédé selon la revendication 1 caractérisé en ce que l'on utilise en tant que catalyseurs d'hydrogénation des catalyseurs au nickel et/ou au cobalt, de préférence du nickel de Raney ou du cobalt de Raney.
